(19) Europäisches Patentamt
European Patent Office
Office européen des brevets

(11) **EP 2 104 486 B2**

(12) **NOUVEAU FASCICULE DE BREVET EUROPEEN**
Après la procédure d'opposition

(45) Date de publication et mention de la décision concernant l'opposition:
**14.04.2021 Bulletin 2021/15**

(45) Mention de la délivrance du brevet:
**29.01.2014 Bulletin 2014/05**

(21) Numéro de dépôt: **07858616.1**

(22) Date de dépôt: **17.10.2007**

(51) Int Cl.:
***A61K 8/34*** *(2006.01)* ***A61K 8/35*** *(2006.01)*
***A61K 8/49*** *(2006.01)* ***A61Q 5/06*** *(2006.01)*

(86) Numéro de dépôt international:
**PCT/FR2007/052193**

(87) Numéro de publication internationale:
**WO 2008/047055 (24.04.2008 Gazette 2008/17)**

# (54) UTILISATION DE COLORANTS NATURELS POUR LA COLORATION DES CHEVEUX HUMAINS

VERWENDUNG NATÜRLICHER FARBSTOFFE ZUR FÄRBUNG VON MENSCHLICHEM HAAR

USE OF NATURAL DYES FOR DYING HUMAN HAIR

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **17.10.2006 FR 0654319**
**01.11.2006 US 855756 P**

(43) Date de publication de la demande:
**30.09.2009 Bulletin 2009/40**

(73) Titulaire: **L'Oréal**
**75008 Paris (FR)**

(72) Inventeur: **DE BONI, Maxime**
**Tokyo (JP)**

(74) Mandataire: **L'Oreal**
**Service D.I.P.I.**
**9, rue Pierre Dreyfus**
**92110 Clichy (FR)**

(56) Documents cités:

**EP-A- 1 022 014** **EP-A- 1 127 566**
**EP-A- 1 454 613** **EP-A1- 0 533 937**
**EP-A1- 1 430 876** **DE-A1- 10 131 385**
**FR-A- 2 543 434** **FR-A- 2 787 707**
**FR-A1- 2 549 721** **FR-A1- 2 787 707**
**GB-A- 1 096 943** **JP-A- H10 265 353**
**US-A- 4 190 064** **US-B2- 7 101 581**

- **DATABASE CA [Online] CHEMICAL ABSTRACTS SERVICE, COLUMBUS, OHIO, US; 27 septembre 2006 (2006-09-27), CHA, DU HWAN ET AL: "Hair care cosmetic composition having double functions of cleaning and dyeing by containing a natural dye henna" XP002507683 extrait de STN Database accession no. 2006:1001982 & KR 2005 109 006 A (KOLMAR CO., LTD., S. KOREA) 17 novembre 2005 (2005-11-17)**
- **STAINSFILE: "Orcein"[Online] 15 mai 2006 (2006-05-15), XP002436770 Extrait de l'Internet: URL:http://web.archive.org/web/20060515092427/http://stainsfile.info/StainsFile/dyes/orcein.htm> [extrait le 2007-06-08]**
- **"HANSEN SOLUBILITY PARAMETERS OF SOLVENTS" INDUSTRIAL SOLVENTS HANDBOOK, XX, XX, 1 janvier 1996 (1996-01-01), pages 35-56, XP000944977**

Remarques:
Le dossier contient des informations techniques présentées postérieurement au dépôt de la demande et ne figurant pas dans le présent fascicule.

EP 2 104 486 B2

## Description

L'invention a pour objet l'utilisation de colorants naturels pour la coloration des cheveux humains.

Il est connu de teindre les fibres kératiniques et en particulier les cheveux humains avec des compositions tinctoriales contenant des précurseurs de colorant d'oxydation, appelés généralement bases d'oxydation, tels que des ortho ou paraphénylènediamines, des ortho ou paraaminophénols et des composés hétérocycliques. Ces bases d'oxydation sont en général associées à des coupleurs. Ces bases et ces coupleurs sont des composés incolores ou faiblement colorés qui, associés à des produits oxydants, peuvent donner naissance par un processus de condensation oxydative à des composés colorés.

Ce type de coloration par oxydation permet d'obtenir des colorations permanentes mais il entraîne une dégradation des fibres kératiniques par l'utilisation d'agents oxydants.

Par ailleurs, il est connu de teindre les fibres kératiniques et en particulier les cheveux humains, avec des compositions de teinture contenant des colorants directs. Les colorants classiques qui sont utilisés sont en particulier des colorants du type nitrés benzéniques, anthraquinoniques, nitropyridiniques, azoïques, azoïques, xanthéniques, acridiniques, aziniques, triarylméthane ou des colorants naturels. Ces colorants peuvent être non ioniques, anioniques, cationiques ou amphotères. Ces colorants sont des molécules colorées et colorantes ayant une affinité pour les fibres kératiniques.

Ces compositions contenant un ou plusieurs colorants directs sont appliquées sur les fibres kératiniques pendant un temps nécessaire à l'obtention de la coloration désirée, puis rincées.

Les colorations qui en résultent sont des colorations particulièrement chromatiques qui sont cependant temporaires ou semi-permanentes car la nature des interactions qui lient les colorants directs à la fibre kératinique, et leur désorption de la surface et/ou du coeur de la fibre sont responsables de leur faible puissance tinctoriale et de leur mauvaise tenue aux lavages ou à la transpiration.

Le document FR 2 549 721 décrit une coloration à partir de colorants naturels. Le procédé de teinture des cheveux décrit dans ce document nécessite cependant une étape de prétraitement avec des sels métalliques pour obtenir une bonne résistance de la coloration résultante.

Le but de la présente invention est de fournir de nouvelles compositions pour la teinture des cheveux humains qui respectent la nature des cheveux et présentent des teintures puissantes, peu sélectives et résistantes, capables d'engendrer de nouveaux colorants puissants pouvant donner des nuances variées.

Ce but est atteint avec la présente invention qui a pour objet un procédé de coloration des fibres kératiniques, notamment les cheveux humains tel que défini à la revendication 1.

Par colorants naturels, on entend tout colorant ou précurseur de colorant ayant une occurrence naturelle et produit soit par extraction (et éventuellement purification) depuis une matrice végétale éventuellement en présence de composés naturels tels que la cendre, l'ammoniac, soit par synthèse chimique.

Les colorants naturels sont choisis parmi les orcéines. On peut également utiliser les extraits ou décoctions contenant ces colorants naturels.

La composition de l'invention comprend une ou plusieurs orcéïnes. Selon une variante, la composition comprend une ou plusieurs orcéïnes et un ou plusieurs colorants additionnels naturels choisis parmi la curcumine, la chlorophyiline, l'isatine, le sorgho et le carmin cochenille

Les orcéïnes sont des colorants obtenus à partir d'extrait d'un lichen, notamment l'orseille et éventuellement modifiés chimiquement notamment par action de l'ammoniac.

Les orcéïnes correspondent notamment à la formule (I) suivante :

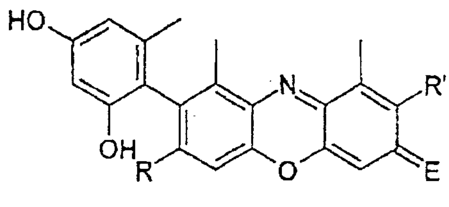

(I)

dans lesquelles R' représente un atome d'hydrogène, un radical benzénique substitué par un radical hydroxy ou alkyle, R représente NH2 ou OH et E représente un atome d'oxygène ou un radical NH.

Selon un mode de réalisation particulier, R' est choisi parmi l'hydrogène,

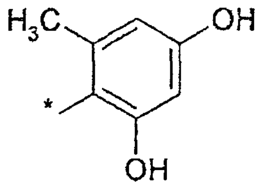 (a) ou 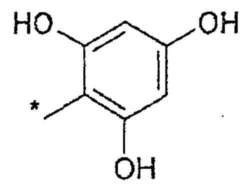 (b)

* représentant la liaison covalente reliant le radical au reste de la formule (I)

A titre d'exemple, on peut citer pour les orcéïnes de formule (I) l'α-amino-orcéïne lorsque R' représente un atome d'hydrogène, E représente l'atome d'oxygène et R un radical NH2, l'α-hydroxy-orcéïne lorsque R' représente un atome d'hydrogène, E représente l'atome d'oxygène, et R un radical hydroxy.

Lorsque R' représente (a), on peut citer la β-amino-orcéïne lorsque R représente NH2 et E un atome d'oxygène, la β-hydroxy-orcéïne lorsque R représente OH et E un atome d'oxygène, la β-amino-orcéïnimine lorsque R représente NH2 et E représente NH.

Lorsque R' représente (b), on peut citer la γ-amino-orcéïne lorsque R représente NH2 et E un atome d'oxygène, la γ-hydroxy-orcéïne lorsque R représente OH et E un atome d'oxygène, la γ-amino-orcéïnimine lorsque R représente NH2 et E représente NH. Selon un mode de réalisation particulier, l'orcéine utile dans la présente invention est l'α-hydroxy-orcéïne.

**[0019]** - Ces orcéïnes sont notamment décrites dans les articles de H Musso suivants: Chemische Berichte, 1959, 92, 751-753, Chemische Berichte 1960, 93, 1782-1788, et Chemische Berichte, 1957, 90, 2190-2194.

A titre d'exemple d'orcéïnes, on peut citer le produit proposé par la société PANREAC sous l'appelation ORCEINE DC.

La quantité de colorants naturels qui peut être utilisée dans les compositions de l'invention est en général comprise entre 0,001 % et 20 % en poids du poids total de la composition, préférentiellement entre 0,1 et 10 %.

Le solvant organique est l'alcool benzylique.

La composition de l'invention comprend généralement une quantité de solvants organiques utiles dans l'invention comprise entre 0,1 et 80 %, de préférence comprise entre 0,5 et 50 % et encore plus préférentiellement entre 1 et 30 % du poids total de la composition.

De préférence, la quantité d'eau est au moins égale à 40 % par rapport au poids total de la composition de coloration. Encore plus préférentiellement, cette quantité d'eau est au moins égale à 70 %.

Selon un mode de réalisation particulier, le milieu approprié à la coloration des fibres kératiniques comprend au moins 70 % d'eau en poids par rapport au poids total de la composition.

La composition de coloration de l'invention peut contenir des solvants organiques additionnels. A titre d'exemple, on peut citer les alcanols inférieurs en $C_1$-$C_4$, tels que l'éthanol et l'isopropanol ; les polyols comme le propylène glycol ou le glycérol et leurs mélanges.

Pour la coloration des fibres kératiniques humaines telles que les cheveux, le milieu de coloration est un milieu cosmétique approprié.

La quantité totale de solvants dans la composition de l'invention peut varier entre 0,1 et 80 % en poids environ par rapport au poids total de la composition, et plus préférentiellement entre 0,5 et 50 % en poids environ et encore plus préférentiellement entre 1 et 30 % du poids total de la composition.

Les compositions de colorations peuvent aussi comprendre un ou plusieurs précurseurs de colorant d'oxydation : une ou plusieurs bases d'oxydation et/ou un ou plusieurs coupleurs. A titre d'exemple, les bases d'oxydation sont choisies parmi les para-phénylènediamines, les bis-phénylalkylènediamines, les para-aminophénols, les ortho-aminophénols, les bases hétérocycliques et leurs sels d'addition.

La ou les bases d'oxydation présentes sont en général présentes en quantité comprise allant de 0,001 à 20 % en poids environ du poids total de la composition tinctoriale, de préférence allant de 0,005 à 6 %.

Les compositions peuvent contenir un ou plusieurs coupleurs conventionnellement utilisés pour la teinture de fibres kératiniques. Parmi ces coupleurs, on peut notamment citer les métaphénylènediamines, les métaaminophénols, les métadiphénols, les coupleurs naphtaléniques, les coupleurs hétérocycliques ainsi que leur sels d'addition.

Le ou les coupleurs sont généralement présents en quantité comprise allant de 0,001 à 20 % en poids environ du poids total de la composition tinctoriale, de préférence allant de 0,005 à 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, rammoniaque, les amines ou les alcanolamines.

Les compositions utiles peuvent aussi contenir un ou plusieurs colorants directs additionnels pouvant notamment être choisis parmi les colorants nitrés de la série benzénique, neutres, acides ou cationiques, les colorants directs azoïques neutres acides ou cationiques, les colorants directs aziniques, les colorants directs triarylméthaniques, les colorants directs indoaminiques.

Selon un mode de réalisation particulier, la composition utile dans l'invention ne contient à titre de colorants que des colorants naturels.

Les compositions utiles peuvent également renfermer divers adjuvants utilisés classiquement dans les compositions pour la teinture des cheveux, tels des polymères anioniques, non-ioniques, cationique, amphotères, zwitterioniques ou leurs mélanges, des agents antioxydants, des agents de pénétration, des agents séquestrants, des parfums, des tampons, des agents dispersants, des agents de conditionnement, des agents filmogènes, des céramides, des agents conservateurs, des agents opacifiants

A titre d'agent de conditionnement, on peut citer les silicones linéaires, cyclique, ramifiées ou non ramifiées, volatiles ou non volatiles. Ces silicones peuvent se présenter sous forme d'huiles, de résines ou de gommes, elles peuvent en particulier être des polyorganosiloxanes insolubles dans le milieu cosmétiquement acceptable.

Les organopolysiloxanes sont définis plus en détail dans l'ouvrage de Walter NOLL "Chemistry and Technology of Silicones" (1968) Academic Press. Ils peuvent être volatils ou non volatils.

Lorsqu'elles sont volatiles, les silicones sont plus particulièrement choisies parmi celles possédant un point d'ébullition compris entre 60° C et 260° C.,

A titre d'agent de conditionnement, on peut aussi utiliser les polymères tels que les polyquaterniums 22, 6, 10, 11, 35 et 37 et le chlorure d'hexadimethrine.

La concentration en agent(s) de conditionnement dans la ou les compositions utiles dans l'invention peut varier de 0,01 à 10% en poids par rapport au poids total de la composition, de préférence de 0,05 à 5% et plus préférentiellement encore de 0,1 à 3%.

Les compositions utiles dans la présente invention peuvent contenir en outre au moins un agent épaississant encore appelé "agents d'ajustement de la rhéologie". Cet agent peut être minéral ou organique.

Les agents épaississants organiques peuvent être choisis parmi les amides d'acides gras (diéthanol- ou monoéthanol-amide de coprah, monoéthanolamide d'acide alkyl éther carboxylique oxyéthyléné), les épaississants polymériques tels que les épaississants cellulosiques (hydroxyéthycellulose, hydroxypropylcellulose, carboxyméthylcellulose), la gomme de guar et ses dérivés (hydroxypropylguar), les gommes d'origine microbienne (gomme de xanthane, gomme de scléroglucane), les homopolymères réticulés d'acide acrylique ou d'acide acrylamidopropanesulfonique et les polymères associatifs neutres, anioniques, amphotères ou cationiques (polymères comprenant des zones hydrophiles, et des zones hydrophobes à chaîne grasse capables, dans un milieu aqueux, de s'associer réversiblement entre eux ou avec d'autres molécules.).

Selon un mode de réalisation particulier, l'épaississant est polymérique et choisi parmi les épaississants cellulosiques (hydroxyéthycellulose, hydroxypropylcellulose, carboxyméthylcellulose), la gomme de guar et ses dérivés (hydroxypropylguar), les gommes d'origine microbienne (gomme de xanthane, gomme de scléroglucane), les homopolymères réticulés d'acide acrylique ou d'acide acrylamidopropanesulfonique.

Les compositions utiles peuvent de plus contenir un ou plusieurs agents tensioactifs.

Les tensioactifs convenant à la mise en oeuvre de la présente invention sont notamment :

(i) Les tensioactif(s) anionique(s) :

A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, $\alpha$-oléfine-sulfonates, paraffine-sulfonates ; les alkyl($C_8$-$C_{24}$) sulfosuccinates, les alkyl($C_6$-$C_{24}$) éthersulfosuccinates, les alkyl($C_6$-$C_{24}$) amidesulfosuccinates ; les alkyl($C_6$-$C_{24}$) sulfoacétates ; les acyl($C_6$-$C_{24}$) sarcosinates et les acyl($C_{6}$-$_{24}$) glutamates. On peut également utiliser les esters d'alkyl($C_6$-$C_{24}$)polyglycosides carboxyliques tels que les alkylglucoside citrates, les alkylpolyglycoside tartrate et les alkylpolyglycoside sulfosuccinates, les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl ($C_6$-$C_{24}$) éther carboxyliques polyoxyalkylénés, les acides alkyl($C_6$-$C_{24}$) aryl éther carboxyliques polyoxyalkylénés, les acides alkyl($C_6$-$C_{24}$) amido éther carboxyliques polyoxyalkylénés et leurs sels, en particulier ceux comportant de 2 à 50 groupements oxyde d'alkylène en particulier d'éthylène, et leurs mélanges.

(ii) les tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178). Ainsi, ils peuvent être notamment choisis parmi (liste non limitative) les alcools, les alpha-diols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène; les esters d'acides gras du sucrose, les esters d'acides gras du polyéthylèneglycol, les alkylpolyglycosides, les dérivés de N-alkyl glucamine, les oxydes d'amines tels que les oxydes d'alkyl ($C_{10}$-$C_{14}$) amines ou les oxydes de N-acylaminopropylmorpholine.

(iii) Tensioactif(s) amphotère(s) ou zwittérionique(s) :

Les agents tensioactifs amphotères ou zwitterioniques peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl ($C_8$-$C_{20}$) bétaïnes, les sulfobétaïnes, les alkyl ($C_6$-$C_{20}$) amidoalkyl ($C_1$-$C_6$) betaïnes ou les alkyl ($C_8$-$C_{20}$) amidoalkyl ($C_1$-$C_6$) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.

A titre d'exemple, on peut citer le cocoamphodiacetate commercialisé sous la dénomination commerciale MIRANOL® C2M concentré par la société RHODIA CHIMIE.

(iv) Les tensioactifs cationiques :

Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

Les quantités d'agents tensioactifs présents dans la composition utile dans le procédé de l'invention peuvent varier de 0,01 à 40% et de préférence de 0,5 à 30% du poids total de la composition.

Le pH de la composition appliquée sur les fibres est en général compris entre 2 et 13, de préférence entre 3 et 8. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante :

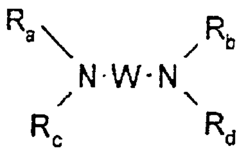

(II)

dans laquelle W est un reste propylène éventuellement substitué par un groupement hydroxyle ou un radical alkyle en

$C_1$-$C_4$ ; $R_a$, $R_b$, $R_c$ et $R_d$, identiques ou différentes, représentent un atome d'hydrogène, un radical alkyle en $C_1$-$C_4$ ou hydroxyalkyle en $C_1$-$C_4$.

Lorsque la composition comprend au moins un précurseur de colorant d'oxydation ou lorsqu'on veut mettre en oeuvre une coloration éclaircissante, un agent oxydant peut être mis en oeuvre.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

Cet agent oxydant peut aussi être présent dans la composition utile dans l'invention ou appliqué indépendamment sous forme d'une composition oxydante.

La composition oxydante peut également renfermer divers adjuvants utilisés classiquement dans les compositions pour la teinture des cheveux et tels que définis précédemment.

Selon l'invention, le temps de pose des compositions est en général compris entre 1 minute et 1 heure.

Le procédé de l'invention consistant à appliquer sur les cheveux humains une composition comprenant un ou plusieurs colorants naturels tels que définis precedemment peut être mis en oeuvre à une température variant entre la température ambiante (20-25°C) et 200°C, de préférence entre la température ambiante et 60°C.

Le procédé de l'invention peut comprendre une étape postérieure de rinçage, ou d'autres étapes ultérieures telles qu'une étape de conditionnement, de mise en forme, etc.

Selon un mode de réalisation particulier, le procédé de l'invention est mis en oeuvre sans prétraitement avec des sels métalliques, notamment sans prétraitement aux sels de cuivre, de zinc, d'aluminium, etc.

Selon un autre mode de réalisation particulier, le procédé de l'invention est un procédé de coloration des cheveux qui comprend comme seules étapes l'application sur les cheveux d'une composition comprenant au moins un colorant naturel tel que défini précédemment pendant un temps suffisant pour l'obtention de la coloration désirée, suivie de façon optionnelle par une étape de rinçage.

A titre d'exemple, ces colorants sont sous forme d'une poudre mélangée à de la sciure d'epicia.

Le procédé est mis en oeuvre à partir d'une composition comprenant une ou plusieurs orcéïnes.

Le ou les colorants naturels, définis precedemment, sont sous forme d'un liquide anhydre dans lequel la ou les colorants sont dissous ou dispersées. Selon une variante, le liquide anhydre utile dans la présente invention est par exemple constitué par les solvants organiques utiles dans la composition de l'invention.

Au moment de l'emploi, la composition anhydre est mélangée avec la seconde composition pour former une composition prête à l'emploi. Une telle composition prête à l'emploi est obtenue avec une facilité de mélange remarquable et permet d'obtenir sur les cheveux une coloration particulièrement homogène de la pointe à la racine des cheveux.

Les exemples qui suivent servent à illustrer l'invention sans toutefois présenter un caractère limitatif.

## EXEMPLES

### Exemple 1

Une composition comprenant 5% d'alcool benzylique, 15% d'éthanol, 0.2% d'acide benzoïque, 1.6% d'hydroxyéthyl cellulose, eau (QSP 100) et 0.5% d'$\alpha$-hydroxy-orcéïne est appliquée 20 minutes à température ambiante sur une mèche de cheveux naturels à 90% de cheveux blancs et sur une mèche de cheveux permanenté à 90% blancs.

Après l'application, les mèches sont rincées, shampouinées et séchées. Elles sont colorées en violine de façon puissante et peu sélective, c'est-à-dire que visuellement la coloration obtenue avec les cheveux naturels et les cheveux permanentés sont très proches. Une série de 12 lavages appliquée sur les deux types de mèches a entraîné une dégradation faible de la coloration (moins de 10%).

### Exemple 2

Une composition comprenant 5% d'alcool benzylique, 15% d'éthanol, 0.2% d'acide benzoïque, de 2 % d'ammoniaque, de 1.6% d'hydroxyéthyl cellulose, eau (QSP 100) et 0.5% d'$\alpha$-hydroxy-orcéïne est mélangée poids pour poids à une formule oxydante de peroxyde d'hydrogène titrant à 20V en eau oxygénée . Ce mélange est appliqué 30 minutes à température ambiante sur une mèche de cheveux naturels à 90% de cheveux blancs et sur une mèche de cheveux permanentés à 90% de cheveux blancs.

Après l'application, les mèches sont rincées, shampouinées et séchées. Elles sont colorées en violine de façon puissante. Les colorations sont très homogènes.

**Exemple 3 (comparatif)**

Les compositions suivante ont été réalisées (quantités exprimées en g%)

| | A (invention) | B (comparatif) |
|---|---|---|
| Alcool benzylique | 5 | - |
| Ethanol | 15 | 20 |
| Acide benzoïque | 0,2 | 0,2 |
| Hydroxyéthyl cellulose | 1,6 | 1,6 |
| Orceine DC (PANREAC FRANCE) | 0,5 | 0,5 |
| Eau | Qs100 | Qs 100 |

Chaque composition est appliquée sur mèches de cheveux naturels à 90% de cheveux blancs (BN), et sur des mèches de cheveux sensibilisés (solubilités alcaline 41,6%)

Chaque mèche traitée est soumis à différents temps de pause :

- Temps de pause 20 mn à température ambiante
- Temps de pause 30 mn à température ambiante

Les mèches sont ensuite rincées, lavées avec un shampooing standard et séchées.

La Coloration des cheveux est évaluée dans le système L*a*b* system, avec un spectrophotometre MINOLTA CM2600-d ®. Dans ce système, L représente l'intensité, plus la valeur de L* est faible, plus la coloration obtenue est intense

| | | L* |
|---|---|---|
| Cheveux BN | Comp A /20 mn | 28,15 |
| | Comp B / 20 mn | 47,81 |
| Cheveux SA 41,6 | Comp A /20 mn | 26,69 |
| | Comp B / 20 mn | 44,49 |
| Cheveux BN | Comp A / 30 mn | 27,51 |
| | Comp B /30 mn | 45,07 |
| Cheveux SA 41,6 | Comp A /30 mn | 27,56 |
| | Comp B /30 mn | 42,59 |

La composition A selon l'invention présente des valeurs de L* inférieures à la composition B, aussi bien à 20 mn qu'à 30 mn de temps de pause. Sur les deux types de cheveux traités et pour les deux temps de pause, la couleur obtenue est plus foncée, plus puissante avec la composition de l'invention composition A comprenant un solvant organique présentant une valeur du paramètre$\delta$H inférieure à 15 (alcool benzylique : valeur de $\delta$H =13,7) qu'avec une composition contenant de l'éthanol pour lequel la valeur de $\delta$H est de 19,4).

**Exemple 4 : Comparatif de procédé avec pré-traitement / sans pré-traitement**

Composition de pré-traitement C (en g%)

| | C |
|---|---|
| Sulfate double d'aluminium et de potassium hydraté ($AlK(SO_4)_2\ 12H_2O$) | 9,48 (= 5,16 en sel non hydraté) |

(suite)

| | C |
|---|---|
| Acide citrique | Qs pH = 3 |
| Eau | Qs 100 g |

Sur des mèches de cheveux naturels à 90% de cheveux blancs et des mèches de cheveux sensibilisés (solubilité alcaline 41,6%), on applique la composition de prétraitement C avec la composition A de l'invention selon l'un des procédés décrits ci-après

Procédé 1 (invention):
coloration avec la composition A
Temps de pause 30 mn
Rinçage, shampooing, séchage

Procédé 2 : (comparatif)
Prétraitement avec la composition C : temps de pause 5 mn
Rinçage à l'eau tiède
Coloration avec la composition A : temps de pause 30 mn
Rinçage, shampooing et séchage

Procédé 3 : (invention)
Coloration avec la composition A
Temps de pause 20 mn
Rinçage, shampooing, séchage

Procédé 4 : (comparatif)
Prétraitement avec la composition C : temps de pause 15 mn
Rinçage à l'eau tiède
Coloration avec la composition A : temps de pause 20 mn
Rinçage, shampooing et séchage

La coloration des mèches est évalué comme dans l'exemple 3 par mesure du L* pour l'évaluation de l'intensité de la coloration. On obtient les résultats suivants :

| | | L* |
|---|---|---|
| **Cheveux BN** | **Procédé 1** | **27,51** |
| | Procédé 2 | 31,66 |
| | **Procédé 3** | **28,15** |
| | Procédé 4 | 31,92 |
| **Cheveux SA 41,6** | **Procédé 1** | **27,56** |
| | Procédé 2 | 29,56 |
| | **Procédé 3** | **26,69** |
| | Procédé 4 | 35,24 |

Les mèches colorées selon le procédé 1 ou 3selon l'invention présentent une valeur de L* inférieure aux mèches colorées selon le procédé 2 ou 4 avec prétraitement aux sels métalliques : la couleur est plus foncée avec un procédé sans prétraitement, et ceci pour un temps de pause de la coloration de 20 et 30 mn.

Les procédés 1 et 2 sont aussi mis en oeuvre sur mèches de cheveux permanentés à 90% de cheveux blancs (BP).

La coloration est évaluée comme décrit précédemment dans le système L*a*b*. Dans ce système, L représente l'intensité, plus la valeur de L* est faible, plus la coloration obtenues est intense La chromaticité est mesurée par les

valeurs a* et b*, a* représentant l'axe rouge/vert et b* l'axe jaune/bleu

La sélectivité de la coloration qui est représentative de l'homogénéité de la coloration le long de la fibre, de la pointe à la racine des cheveux est évaluée selon ΔE, qui est la variation de la couleur entre les cheveux naturels teints et les cheveux permanentés teints, est obtenu à partir de la formule :

$$\Delta E = \sqrt{(L^* - L_o^*)^2 + (a^* - a_o^*)^2 + (b^* - b_o^*)^2}$$

dans laquelle L* représente l'intensité a* et b*, la chromaticité des cheveux naturels teints et $L_0$* représente l'intensité et $a_0$* et $b_0$* la chromaticité des cheveux sensibilisés teints. Plus la valeur de ΔE est faible, plus la sélectivité est faible et la coloration uniforme le long des cheveux.

Les résultats sont reportés dans le tableau ci-dessous :

| | L* | a* | b* | Sélectivité |
|---|---|---|---|---|
| BN procédé 1 | 27,51 | 14,06 | 1,83 | 3,30 |
| BP procédé 1 | 24,24 | 14,42 | 1,61 | |
| BN procédé 2 | 31,66 | 14,81 | 3,27 | 8,59 |
| BP procédé 2 | 23,36 | 12,92 | 4,44 | |

L'homogénéité de la coloration est bien plus uniforme avec le procédé de la présente invention sans prétraitement.

**Revendications**

1. Procédé de coloration des cheveux humains comprenant l'application sur les cheveux sans traitement préalable d'une composition de coloration qui comprend un ou plusieurs colorants naturels choisi parmi les orceines, et l'alcool benzylique pendant un temps suffisant à l'obtention de la coloration désirée, suivie de façon optionnelle par une étape de rinçage.

2. Procédé selon la revendication 1 dans lequel la composition comprend une ou plusieurs orcéines et un ou plusieurs colorants additionnels naturels choisis parmi la curcumine, la chlorophylline, l'isatine, le sorgho et le carmin cochenille.

3. Procédé selon l'une quelconque des revendications 2 dans lequel la ou les orcéines sont choisies parmi les orcéines de formule (**I**) suivantes :

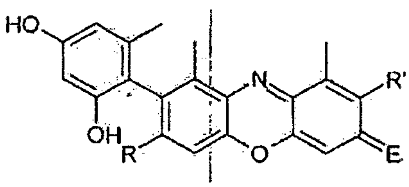
(I)

dans lesquelles R' représente un atome d'hydrogène, un radical benzénique substitué par un radical hydroxy ou alkyle, R représente NH2 ou OH et E représente un atome d'oxygène ou un radical NH.

4. Procédé selon la revendication 3 dans lequel R' représente un atome d'hydrogène, un radical choisi parmi

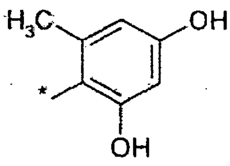 (a) ou 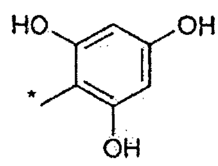 (b)

* représentant la liaison covalente reliant le radical au reste de la formule (I).

5. Procédé selon l'une quelconque des revendications 1 à 4 dans lequel les orcéïnes sont choisies parmi l'a-amino-orcéïne ou l'α-hydroxy-orcéïne.

6. Procédé selon l'une quelconque des revendications 1 à 5 dans lequel les orcéïnes sont choisies parmi la β-amino-orcéïne, la β-hydroxy-orcéïne, la β-amino-orcéinimine

7. Procédé selon l'une quelconque des revendications 1 à 6 dans lequel les orcéïnes sont choisies parmi la γ-amino-orcéïne, la γ-hydroxy-orcéïne, la γ-amino-orcéinimine

8. Procédé selon l'une quelconque des revendications 1 à 6 dans laquelle l'orcéine est l'α-hydroxy-orcéine.

9. Procédé selon l'une quelconque des revendications 1 à 8 dans lequel les colorants naturels sont présents en quantité comprise entre 0,001 et 20 % en poids du poids de la composition.

10. Procédé selon l'une quelconque des revendications précédentes dans lequel la composition comprend de plus un ou plusieurs agents tensio-actifs et/ou un ou plusieurs polymères épaississants.

11. Procédé selon l'une quelconque des revendications précédentes dans lequel la composition comprend au moins 70 % d'eau.

**Patentansprüche**

1. Verfahren zum Färben von menschlichem Haar, bei dem man ohne vorherige Behandlung auf das Haar eine Färbezusammensetzung aufbringt, die einen oder mehrere natürliche Farbstoffe, ausgewählt aus den Orceinen und Benzylalkohol umfasst, über einen Zeitraum, der ausreicht, um zu der gewünschten Färbung zu gelangen, gewünschtenfalls gefolgt von einem Spülschritt.

2. Verfahren nach Anspruch 1, wobei die Zusammensetzung ein oder mehrere Orceine und einen oder mehrere zusätzliche natürliche Farbstoffe, ausgewählt aus Curcumin, Chlorophyllin, Isatin, Sorgho und Cochenill-Karmin umfasst.

3. Verfahren nach einem der Ansprüche 2, wobei das Orcein bzw. die Orceine aus den Orceinen der folgenden Formel (I) ausgewählt sind:

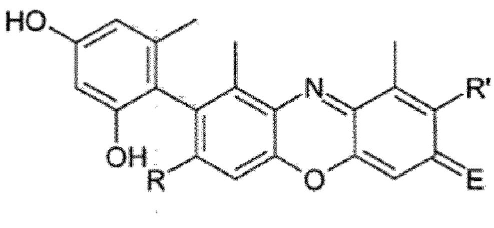 (I)

wobei R' ein Wasserstoffatom, eine Benzolgruppe, die durch eine Hydroxy- oder Alkylgruppe substituiert ist, bedeutet, R NH2 oder OH bedeutet und E ein Sauerstoffatom oder eine NH-Gruppe bedeutet.

4. Verfahren nach Anspruch 3, wobei R' ein Wasserstoffatom oder eine Gruppe, ausgewählt aus

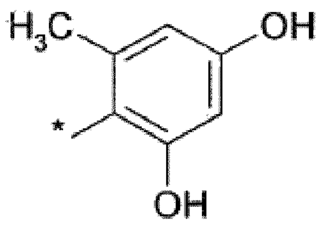 (a) oder 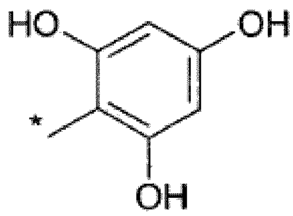 (b)

wobei * die kovalente Bindung, die die Gruppe mit dem Rest der Formel (I) verbindet, bedeutet, bedeutet.

5. Verfahren nach einem der Ansprüche 1 bis 4, wobei die Orceine aus α-Aminoorcein oder α-Hydroxyorcein ausgewählt sind.

6. Verfahren nach einem der Ansprüche 1 bis 5, wobei die Orceine aus β-Aminoorcein, β-Hydroxyorcein und β-Aminoorceinimin ausgewählt sind.

7. Verfahren nach einem der Ansprüche 1 bis 6, wobei die Orceine aus γ-Aminoorcein, γ-Hydroxyorcein und γ-Aminoorceinimin ausgewählt sind.

8. Verfahren nach einem der Ansprüche 1 bis 6, wobei es sich bei dem Orcein um α-Hydroxyorcein handelt.

9. Verfahren nach einem der Ansprüche 1 bis 8, wobei die natürlichen Farbstoffe in einer Menge zwischen 0,001 und 20 Gew.-% des Gewichts der Zusammensetzung vorliegen.

10. Verfahren nach einem der vorhergehenden Ansprüche, wobei die Zusammensetzung weiterhin ein oder mehrere Tenside und/oder ein oder mehrere verdickende Polymere umfasst.

11. Verfahren nach einem der vorhergehenden Ansprüche, wobei die Zusammensetzung mindestens 70% Wasser umfasst.

## Claims

1. Method of dyeing human hair comprising the application to the hair, without pretreatment, of a dyeing composition that comprises one or more natural dyes chosen from orceins, and benzyl alcohol, for a sufficient time to obtain the desired colouring, optionally followed by a rinsing step.

2. Method according to Claim 1, in which the composition comprises one or more orceins and one or more additional natural dyes chosen from curcumin, chlorophyllin, isatin, sorghum and cochineal carmine.

3. Method according to any one of Claims 2, in which the orcein or orceins are chosen from the orceins of formula (I) below:

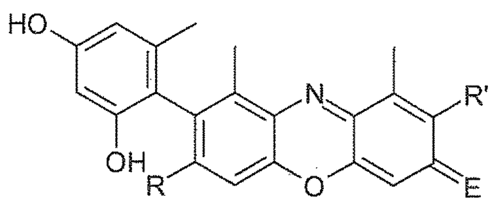 (I)

in which R’ represents a hydrogen atom, a benzene radical substituted by a hydroxyl or alkyl radical, R represents $NH_2$ or OH and E represents an oxygen atom or an NH radical.

4. Method according to Claim 3, in which R’ represents a hydrogen atom or a radical chosen from:

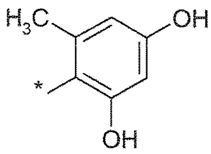 (a) or 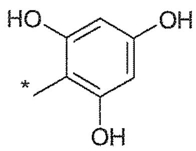 (b)

* representing the covalent bond connecting the radical to the rest of the formula (I).

5. Method according to any one of Claims 1 to 4, in which the orceins are chosen from α-aminoorcein or α-hydroxyorcein.

6. Method according to any one of Claims 1 to 5, in which the orceins are chosen from β-aminoorcein, β-hydroxyorcein or β-aminoorceinimine.

7. Method according to any one of Claims 1 to 6, in which the orceins are chosen from γ-aminoorcein, γ-hydroxyorcein or γ-aminoorceinimine.

8. Method according to any one of Claims 1 to 6, in which the orcein is α-hydroxyorcein.

9. Method according to any one of Claims 1 to 8, in which the natural dyes are present in an amount between 0.001 and 20% by weight of the weight of the composition.

10. Method according to any one of the preceding claims, in which the composition comprises, in addition, one or more surfactants and/or one or more thickening polymers.

11. Method according to any one of the preceding claims, in which the composition comprises at least 70% water.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2549721
- US 2528378 A
- US 2781354 A


### Littérature non-brevet citée dans la description


- **H MUSSO.** *Chemische Berichte,* 1959, vol. 92, 751-753
- **H MUSSO.** *Chemische Berichte,* 1960, vol. 93, 1782-1788
- **H MUSSO.** *Chemische Berichte,* 1957, vol. 90, 2190-2194
- **WALTER NOLL.** Chemistry and Technology of Silicones. Academic Press, 1968
- **M.R. PORTER.** Handbook of Surfactants. 1991, 116-178
- dénominations Amphocarboxyglycinates et Amphocarboxypropionates. le dictionnaire CTFA. 1982